# EUROPEAN PATENT APPLICATION

(11) **EP 2 110 066 A1**
(43) Date of publication of application: **21.10.2009**
(21) Application number: 08703773.5
(22) Date of filing: 24.01.2008
(51) Int. Cl.: A61B 1/00

(54) **DEVICE FOR CHECKING FOR LUMEN PASSAGE AND METHOD OF CHECKING FOR LUMEN PASSAGE**

(30) Priority: 06.02.2007 JP 2007026323
(71) Applicant: Olympus Medical Systems Corporation, Tokyo 151-0072 (JP)
(72) Inventor: TAKIZAWA, Hironobu, Tokyo 151-0072 (JP)
(74) Representative: von Hellfeld, Axel
(86) International application number: PCT/JP2008/050944
(87) International publication number: WO 2008/096606

(57) **Abstract**

An object of the present invention is to make it possible to easily check a passability of a capsule medical device through a lumen of a subject until when the capsule medical device reaches a target organ within the subject. A pretest capsule (1) according to the present invention is an example of a lumen passability checking device that checks a passability of the capsule medical device inserted into a body of the subject, and the pretest capsule (1) includes an outer case (2) having an external diameter substantially equal to that of the capsule medical device, and a pigment (3) contained in the outer case (2). The outer case (2) is dissolved by a specific substance existing within large intestine of the subject. The pigment (3) is discharged from the outer case (2) to the inside of the subject along dissolution of the outer case (2), and is excreted from the body together with a bodily waste of the subject.

## Description

### TECHNICAL FIELD

The present invention relates to a lumen passability checking device inserted into an organ of a subject to check beforehand whether a capsule medical device inserted into the organ of the subject such as a patient can pass through the inside of the lumen of the subject (that is, a passability of a capsule medical device through a lumen), and relates to a lumen passability checking method.

### BACKGROUND ART

Capsule endoscopes having an imaging function and a radio communication function have been produced in the field of endoscope in recent years. A subject such as a patient ingests the capsule endoscope from his mouth to perform a capsule endoscope examination to observe (examine) the inside of an organ. After the patient ingests the capsule, the capsule endoscope moves within organs such as stomach and small intestine by a peristaltic movement or the like until when the capsule endoscope is naturally excreted from the subject. During this period, the capsule endoscope sequentially captures images within the organ of the subject (hereinafter, also referred to as "in-vivo images") at 0.5-second intervals, for example.
The capsule endoscope sequentially radio-transmits the captured in-vivo images to a receiving device carried by the subject.

The receiving device carried by the subject sequentially receives the in-vivo images that are radio-transmitted by the capsule endoscope, and sequentially accumulates the in-vivo images within a recording medium of the receiving device. Thereafter, the recording medium that stores an in-vivo image group of the subject is detached from the receiving device, and is attached to a predetermined image display device. The image display device acquires the in-vivo image group of the subject via the recording medium, and displays the in-vivo image group of the subject on a display. A doctor or nurse observes the in-vivo images displayed in the image display device, and performs a diagnosis of the subject.

When a stenosis part exists within an organ (within a lumen) of a subject, a capsule endoscope taken into the subject to perform the capsule endoscope examination tends to be stagnated at the stenosis part. Therefore, before performing the capsule endoscope examination on the subject, a doctor or nurse needs to check a passability of the capsule endoscope, to be taken into the subject, through a lumen. As a device that checks a passability of the capsule endoscope through the lumen, there has been a device formed by an external coating and an internal filling material, and when the device stagnates at a stenosis part within the lumen, the device collapses the external coating and discharges the internal filling material to the inside of the lumen, and marks (colors) the stenosis part by a marker included in the discharged internal filling material (see Patent Document 1). The doctor or nurse checks appearance of the marker in a bodily waste of the subject (coloring of the bodily waste), thereby recognizing the existence of the stenosis part within the lumen of the subject.

Patent Document 1: Published Japanese translation of a PCT application No. 2005-508668

### DISCLOSURE OF INVENTION

### PROBLEM TO BE SOLVED BY THE INVENTION

However, according to the conventional technique described in the Patent Document 1, while it is possible to confirm that a stenosis part through which a capsule medical device such as a capsule endoscope cannot easily pass exists in a lumen of a subject, it is not easy to determine whether the capsule medical device safely passes through the inside of the lumen and reaches the target organ (the organ to be examined) within the subject. That is, according to the conventional technique, to check the passability of the capsule medical device through the lumen until when the device reaches the organ to be examined, the stenosis part within the lumen needs to be detected by using an apparatus at the outside of the subject such as an RF-ID communication apparatus or an X-ray apparatus.

The present invention has been achieved in view of the above circumstances, and an object of the invention is to provide a lumen passability checking device and a lumen passability checking method capable of easily checking a passability of a capsule medical device through a lumen until when the capsule medical device reaches a target organ within a subject.

### MEANS FOR SOLVING PROBLEM

To solve the problem described above and achieve the object, a lumen passability checking device according to the present invention, which checks a lumen passability of a capsule medical device to be inserted into a body of a subject, includes: an outer structure having an external diameter substantially equal to that of the capsule medical device and having at least a part dissolved by a specific substance existing in a target organ within the subject; and a visually checkable identifier contained in the outer structure and discharged from the outer structure to inside of the body of the subject along dissolution of the outer structure.

In the lumen passability checking device according to the present invention as set forth in the invention described above, the outer structure is collapsed by the specific substance.

In the lumen passability checking device according to the present invention as set forth in the invention described above, the outer structure includes: a first dissoluble unit dissolved by the specific substance; and a second dissoluble unit dissolved by a substance in the body of the subject after a lapse of time longer than a time necessary for the capsule medical device to reach the target organ, wherein the outer structure is shrink-deformed or collapsed by dissolution of the second dissoluble unit.

In the lumen passability checking device according to the present invention as set forth in the invention described above, the outer structure includes: a first dissoluble unit dissolved by the specific substance; and a second dissoluble unit dissolved by another specific substance different from the specific substance, wherein the outer structure is shrink-deformed or collapsed by dissolution of the second dissoluble unit.

In the lumen passability checking device according to the present invention as set forth in the invention described above, the lumen passability checking device further includes another visually checkable identifier presenting a state different from that of the identifier, wherein the outer structure includes: a first dissoluble unit containing the identifier and dissolved by the specific substance; and a second dissoluble unit containing the another identifier and dissolved by another specific substance different from the specific substance, and wherein the identifier is discharged from the outer structure to the inside of the body of the subject along dissolution of the first dissoluble unit, and the another identifier is discharged from the outer structure to the inside of the body of the subject along dissolution of the second dissoluble unit.

In the lumen passability checking device according to the present invention as set forth in the invention described above, the first dissoluble unit forms the external diameter of the outer structure and maintains the external diameter before and after dissolution of the second dissoluble unit.

In the lumen passability checking device according to the present invention as set forth in the invention described above, the identifier is a reflection particle that reflects visible light or a pigment.

In the lumen passability checking device according to the present invention as set forth in the invention described above, the identifier generates a visually checkable change by reacting with a substance within large intestine.

In the lumen passability checking device according to the present invention as set forth in the invention described above, the identifier generates a visually checkable change by reacting with a predetermined reagent.

In the lumen passability checking device according to the present invention as set forth in the invention described above, the identifier is substantially transparent having no color before reaction.

Further, a lumen passability checking method according to the present invention for checking a lumen passability of a capsule medical device to be inserted into a body of a subject, includes: ingesting for having the subject ingested a lumen passability checking device that includes a visually checkable identifier within an outer structure having an external diameter substantially equal to that of the capsule medical device and having at least a part dissolved by a specific substance existing in a target organ within the subject; visually checking a bodily waste of the subject excreted during a period from when the subject ingests the lumen passability checking device until when a predetermined time elapses; and determining that the capsule medical device passes through inside of the lumen of the subject and reaches the target organ, when the identifier is visually checked in the bodily waste of the subject at the visually checking.

In the lumen passability checking method according to the present invention as set forth in the invention described above, at the ingesting, the subject ingests the lumen passability checking device that contains another identifier presenting a state different from that of the identifier in a dissoluble unit that is a part of the outer structure and is dissolved by a substance within the body different from the specific substance, and contains the identifier in a dissoluble unit that is a remaining part of the outer structure and is dissolved by the specific substance, and at the determining, when the identifier and the another identifier are visually checked in the bodily waste of the subject at the visually checking, it is determined that the capsule medical device passes through inside of the subject and reaches the target organ.

In the lumen passability checking method according to the present invention as set forth in the invention described above, at the determining, when the another identifier is visually checked without visually checking the identifier at the visually checking, it is determined that the capsule medical device reaches the organ within the subject in which the substance within the subject exists.

In the lumen passability checking method according to the present invention as set forth in the invention described above, at the visually checking, a plurality of bodily wastes excreted from the subject are visually checked sequentially.

In the lumen passability checking method according to the present invention as set forth in the invention described above, the method further includes cleaning the inside of the lumen of the subject to which the identifier is discharged, when it is determined at the determining that the capsule medical device passes through the inside of the lumen of the subject and reaches the target organ.

In the lumen passability checking method according to the present invention as set forth in the invention described above, the method further includes dissolving-liquid ingesting at which the subject ingests a dissolving liquid for dissolving at least the part of the outer structure when it is determined at the determining that the capsule medical device does not reach the target organ.

Furthermore, a lumen passability checking method according to the present invention for checking a lumen passability of a capsule medical device to be inserted into a body of a subject, includes: ingesting for having the subject ingested a lumen passability checking device that includes an identifier within an outer structure having an external diameter substantially equal to that of the capsule medical device and having at least a part dissolved by a specific substance existing in a target organ within the subject; visually checking for mixing a reagent into a bodily waste of the subject excreted during a period from when the subject ingests the lumen passability checking device until when a predetermined time elapses, thereby making the identifier in the bodily waste visible, and visually checking the visible identifier; and determining that the capsule medical device passes through inside of the lumen of the subject and reaches the target organ, when the visible identifier is visually checked in the bodily waste of the subject at the visually checking.

In the lumen passability checking method according to the present invention as set forth in the invention described above, the method further includes dissolving-liquid ingesting at which the subject ingests a dissolving liquid for dissolving at least the part of the outer structure when it is determined at the determining that the capsule medical device does not reach the target organ.

Still further, a lumen passability checking device according to the present invention, which checks a lumen passability of a capsule medical device to be inserted into a body of a subject, includes: an outer structure having an external diameter substantially equal to that of the capsule medical device and having at least a part dissolved by a specific substance existing in a target organ within the subject; and an identifier contained in the outer structure and discharged from the outer structure to inside of the body of the subject along dissolution of the outer structure, wherein the identifier can be checked by a smell.

Furthermore, a lumen passability checking method according to the present invention for checking a lumen passability of a capsule medical device to be inserted into a body of a subject, includes: ingesting for having the subject ingested a lumen passability checking device that includes an identifier that can be checked by a smell within an outer structure having an external diameter substantially equal to that of the capsule medical device and having at least a part dissolved by a specific substance existing in a target organ within the subject; checking presence of the smell of the identifier from a bodily waste of the subject excreted during a period from when the subject ingests the lumen passability checking device until when a predetermined time elapses; and determining that the capsule medical device passes through inside of the lumen of the subject and reaches the target organ, when the smell of the identifier is checked from the bodily waste of the subject at the visually checking.

### EFFECT OF THE INVENTION

The lumen passability checking device according to the present invention is configured as follows by forming an outer structure having an external diameter substantially equal to that of a capsule medical device inserted into the body of a subject, by using a member that is dissolved by a specific substance existing within a target organ, and a visually checkable identifier such as a pigment is filled into the outer structure. The identifier is discharged from the outer structure when the outer structure is dissolved within the target organ as a reach target region of the capsule medical device. Therefore, a passability of the capsule medical device through the lumen until when the device reaches the target organ within the subject can be easily checked by a simple structure, without checking the presence of the outer structure or the identifier by using an apparatus at the outside of the subject such as an RF-ID communication apparatus or an X-ray apparatus.

The lumen passability checking method according to the present invention is provided as follows. A subject orally ingests an outer structure containing a visually checkable identifier. Thereafter, a bodily waste excreted from the subject is visually checked during a period until when a predetermined time elapses. Based on a result of the visual check as to whether the bodily waste of the subject includes the identifier (for example, whether the bodily waste of the subject is colored by a pigment), it is determined whether the capsule medical device reaches a target organ within a predetermined time since when the subject orally ingests the capsule medical device. Therefore, a passability of the capsule medical device through the lumen until when the device reaches the target organ within the subject can be easily checked, based on the result of the visual check of the bodily waste of the subject, without checking the presence of the outer structure or the identifier by using an apparatus at the outside of the subject such as an RF-ID communication apparatus or an X-ray apparatus.

### BRIEF DESCRIPTION OF DRAWINGS

FIG. 1 is a schematic diagram of a configuration example of a pretest capsule according to a first embodiment of the present invention.
FIG. 2 is a schematic diagram of a state that a pretest capsule according to the present invention is orally ingested.
FIG. 3 is a flowchart for exemplifying a lumen passability checking method according to the first embodiment of the present invention.
FIG. 4 is a schematic diagram for exemplifying a state that a pretest capsule stagnates at a stenosis part within a lumen of a subject.
FIG. 5 is a schematic diagram for exemplifying a state that a pigment is discharged from a pretest capsule having reached a target organ of the subject.
FIG. 6 is a vertical cross-sectional schematic diagram of a configuration example of a pretest capsule according to a first modification of the first embodiment of the present invention.
FIG. 7 is a schematic diagram of a configuration example of a pretest capsule according to a second modification of the first embodiment of the present invention.
FIG. 8 is a schematic diagram of a configuration example of a pretest capsule according to a second embodiment of the present invention.
FIG. 9 is a schematic diagram of a state that an outer structure of a pretest capsule is broken into a body unit and end units.
FIG. 10 is a flowchart for exemplifying a lumen passability checking method according to the second embodiment of the present invention.
FIG. 11 is a schematic diagram for explaining shrink deformation of a pretest capsule having stagnated at a stenosis part within a lumen.
FIG. 12 is a schematic diagram of a configuration example of a pretest capsule according to a third embodiment of the present invention.
FIG. 13 is a cross-sectional schematic diagram for exemplifying a vertical cross-sectional configuration of the pretest capsule according to the third embodiment of the present invention.
FIG. 14 is a flowchart for exemplifying a lumen passability checking method according to the third embodiment of the present invention.
FIG. 15 is a schematic diagram of a state that a pretest capsule having stagnated at a stenosis part within small intestine discharges a pigment.
FIG. 16 is a schematic diagram of a configuration example of a pretest capsule according to a first modification of the third embodiment of the present invention.
FIG. 17 is a schematic diagram of a configuration example of a pretest capsule according to a second modification of the third embodiment of the present invention.

### EXPLANATIONS OF LETTERS OR NUMERALS

1, 5, 11, 21, 31 Pretest capsule
2, 6, 14, 22, 32 Outer case
3, 4 Pigment
6a Internal dissoluble unit
6b External dissoluble unit
12a to 12c, 32a Large-intestine dissoluble unit
13a to 13c Limonene dissoluble unit
22a Body unit
22b, 22c Lid
32b Small-intestine dissoluble unit
K Subject
P Stenosis part

### BEST MODE(S) FOR CARRYING OUT THE INVENTION

Exemplary embodiments of a lumen passability checking device and a lumen passability checking method according to the present invention will be described below in detail with reference to the accompanying drawings. In the following explanations, a capsule device (a pretest capsule) is shown as an example of the lumen passability checking device according to the present invention. A check (test) of a lumen passability of a capsule medical device until when the device reaches large intestine (an example of a target organ) of a subject by using the pretest capsule is described. However, the present invention is not limited by this embodiment.

### (First embodiment)

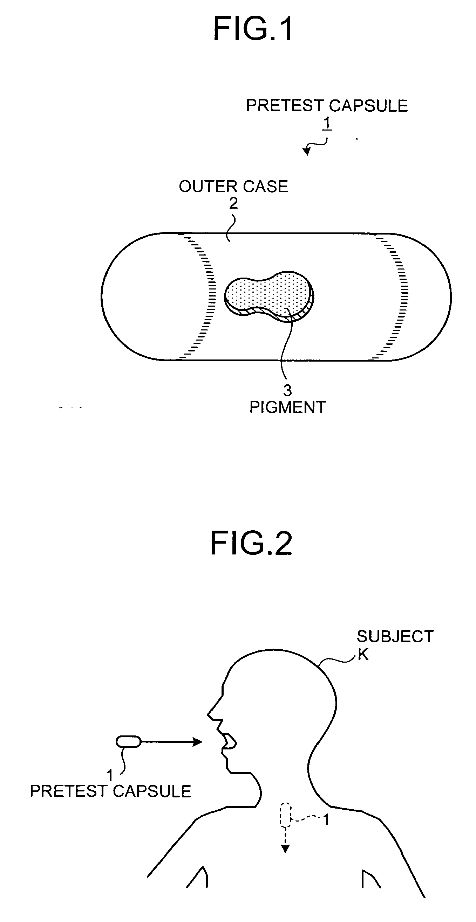

FIG. 1 is a schematic diagram of a configuration example of a pretest capsule according to a first embodiment of the present invention. To explain an internal configuration of the pretest capsule according to the first embodiment, FIG. 1 depicts the pretest capsule in a state that a part of an outer structure is broken. As shown in FIG. 1, a pretest capsule 1 according to the first embodiment has an outer case 2, and the outer case 2 has a pigment 3 therein.

The outer case 2 functions as an outer structure of the pretest capsule 1, and is formed to have a size insertable into the body of the subject. Specifically, the outer case 2 has an external diameter substantially equal to that of a capsule medical device inserted into a body of a subject. Preferably, the outer case 2 is formed in an external shape (a capsule shape) similar to that of the capsule medical device. Even when an external pressure is applied to the outer case 2 by a peristaltic movement of the organ, the outer case 2 maintains an external diameter substantially equal to that of the capsule medical device (further, an external shape similar to that of the capsule medical device).

Further, the outer case 2 is dissolved by a specific substance that exists in a target organ within the subject (that is, the organ as a reach target region of the capsule medical device). Specifically, when the target organ within the subject is large intestine, the outer case 2 is formed by using an enteric member (hereinafter, referred to as "specific enteric member") such as chitosan, which is dissolved by a specific substance existing in the large intestine. The outer case 2 can be formed by only the specific enteric member, or can be an outer case having a specific enteric member coated on an external wall of a structure formed by a general enteric member such as gelatin (that is, a member that is dissolved in both small intestine and large intestine). Such a specific substance within the large intestine that dissolves the specific enteric member is a specific bacterium (a bacterium within large intestine) or a chemical substance (such as an enzyme generated by the bacterium within the large intestine) existing within the large intestine, for example.

The pigment 3 is an edible dye that is safe for a living body, and is contained in the outer case 2 described above. The pigment 3 is discharged to the inside of the body of the subject when the outer case 2 is dissolved. Specifically, along the dissolution of the outer case 2 by the specific substance within the large intestine, the pigment 3 is discharged from the outer case to within the large intestine. Thereafter, the pigment 3 is excreted from the body together with a bodily waste (urine or solid waste). In this case, the pigment 3 colors the bodily waste, thereby notifying the outside (a doctor or nurse, or the subject who ingests the pretest capsule 1 into the body) that the pretest capsule 1 has reached the large intestine. The pigment 3 functions as a visually checkable identifier to identify whether the capsule medical device inserted into the body of the subject safely passes through the inside of the lumen and reaches the target organ (the large intestine, for example).

The pigment 3 can be an edible dye having a desired color such as a red food coloring, and has preferably a color other than that having a possibility of being included in the bodily waste of the subject (such as red, white, black, green, and yellow), that is, an edible dye of a color such as blue or aqua that is not usually included in the bodily waste. Alternatively, the pigment 3 can be that having biocompatibility or indigestibility, without being limited to the edible dye. A mode of the pigment 3 can be a powder (a particle shape) or a liquid shape (for example, the pigment dissolved in a liquid such as water or a normal saline solution).

Before inserting (specifically, before orally ingesting) the capsule medical device into the body, a subject K orally ingests the pretest capsule 1 having this configuration, as shown in FIG. 2, for example. The pretest capsule 1 orally ingested by the subject K advances within the lumen of the subject K by a peristaltic movement or the like. When there is no stenosis part within the lumen of the subject K, the pretest capsule 1 reaches a reach target region (large intestine, for example) of the capsule medical device to be inserted into the body of the subject K, and discharges the pigment 3 to the inside of the target organ as the reach target region. On the other hand, when a stenosis part exists within the lumen of the subject K, the pretest capsule 1 stagnates at this stenosis part. A user such as a doctor or nurse checks (tests) in advance, by using this pretest capsule 1, the passability of the capsule medical device through the lumen until when the capsule medical device reaches the large intestine of the subject K.

For this capsule medical device, there can be listed a capsule endoscope having an imaging function and a radio communication function, a capsule pH-measuring device for measuring pH within a living body, a capsule drug dosing device having a function of dispersing or injecting a drug into a living body, and a capsule collection device for collecting a substance from within a living body.

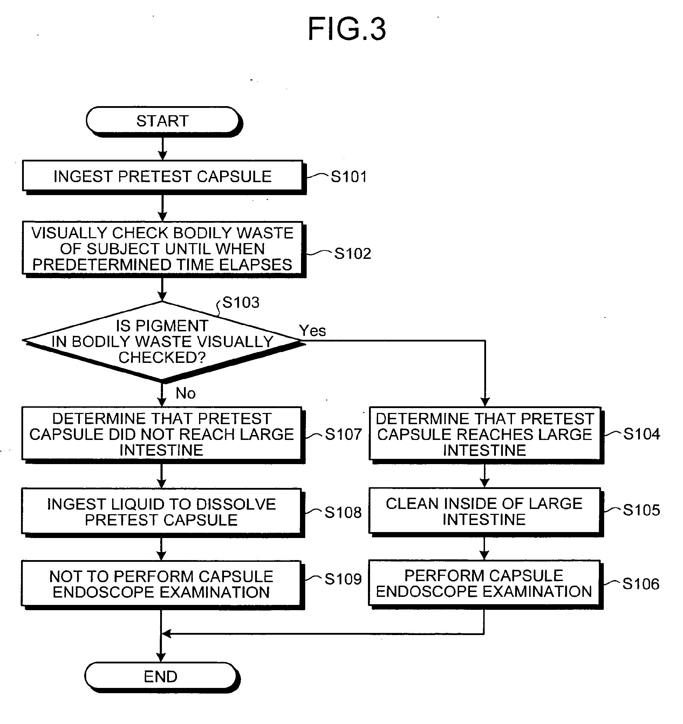

A method of checking a passability of the capsule medical device through the lumen by using the pretest capsule 1 according to the first embodiment of the present invention is described next. FIG. 3 is a flowchart for exemplifying the lumen passability checking method according to the first embodiment of the present invention. The lumen passability checking method according to the first embodiment of the present invention is explained below by exemplifying a case of checking beforehand a passability of the capsule endoscope through the lumen of the subject K before performing a capsule endoscope examination of observing (testing) the inside of the organ by inserting the capsule endoscope into the body.

In FIG. 3, a user such as a doctor or nurse first prompts the subject K to ingest the pretest capsule 1 having an external diameter equal to that of the capsule endoscope to be inserted into the body of the subject K (Step S101). At Step S101, the subject K orally ingests the pretest capsule 1 in a similar manner to that of orally ingesting the capsule endoscope to perform the capsule endoscope examination.

Next, the user visually checks a bodily waste excreted from the subject K during a period from when the subject K ingests the pretest capsule 1 until when a predetermined time elapses (Step S102). Alternatively, the subject K can personally visually check a bodily waste and report a result of the visual check to the user or the like. After the subject K ingests the pretest capsule 1, the pretest capsule 1 advances within the lumen of the subject K by a peristaltic movement or the like. When there is no stenosis part within the lumen of the subject K, the pretest capsule 1 reaches a reach target region (large intestine, for example) of the capsule endoscope, and discharges the pigment 3 to the inside of the large intestine. On the other hand, when a stenosis part exists within the lumen of the subject K, the pretest capsule 1 stagnates at this stenosis part. At Step S102, the user checks whether the pigment 3 can be visually checked in the bodily waste excreted from the subject K by the time when a predetermined time elapses (that is, whether the bodily waste of the subject K is colored by the pigment 3). The predetermined time is longer (20 hours, for example) than the time necessary for the capsule endoscope to reach the reach target region (large intestine) by passing through the inside of the lumen after the subject K orally ingests the capsule endoscope.

At Step S102, the user can visually check the bodily waste excreted from the subject K after a lapse of the predetermined time since the subject K ingests the pretest capsule 1, or can sequentially visually check plural bodily wastes excreted from the subject K at plural times during a period until when the predetermined time elapses.

When the bodily waste of the subject K is colored by the pigment 3, that is, when the user visually checks the pigment 3 in the bodily waste of the subject K at Step S102 (YES at Step S103), the user determines that the pretest capsule 1 has reached the large intestine of the subject K within the predetermined time (Step S104). Based on a result of this determination, the user determines that when the subject K orally ingests the capsule endoscope, this capsule endoscope reaches the large intestine within the predetermined time by safely passing through the inside of the lumen (that is, there is no problem in the lumen passability of the capsule endoscope regarding the subject K).

Next, the user prompts the subject K to ingest a predetermined cleaning agent having no problem in the passability of the capsule endoscope through the lumen, thereby cleaning the inside of the large intestine of the subject K (Step S105). In this case, the pigment 3 of the pretest capsule 1 is in the state of being discharged to the inside of the large intestine of the subject K. When this state is kept as it is, there can be a problem in subsequently performing the capsule endoscope examination. Therefore, the user prompts the subject K to ingest the predetermined cleaning agent at Step S105, thereby removing the pigment 3 and the rest of the outer case 2 remaining in the large intestine of the subject K. For the cleaning agent, there can be listed a neutralizer of the pigment 3, a purgative drug, and an intestinal cleaning liquid. When a region to be examined by the capsule endoscope is determined as only the small intestine, for example, there is no problem when the pigment 3 remains in the large intestine. In this case, Step S105 of ingesting the cleaning agent can be omitted. Accordingly, the examination becomes more convenient.

After the subject K is checked to have no problem in the passability of the capsule endoscope through the lumen and after the large intestine is cleaned, the user prompts the subject K to orally ingest the capsule endoscope, thereby performing the capsule endoscope examination on the subject K (Step S106). Consequently, a capsule-endoscope lumen-passability checking process to the subject K is completed. When the capsule endoscope examination (Step S106) is started at nine in the morning of a day and also when it takes one hour from a completion of a visual check of the bodily waste until when the subject K ingests the cleaning agent (Step S105), for example, it is preferable that the subject K ingests the pretest capsule 1 twenty hours before, that is, at around the noon of one day before the day of the start of the capsule endoscope examination.

On the other hand, when the user visually confirms at Step S102 that the bodily waste of the subject K is not colored by the pigment 3, that is, when the user does not visually find the pigment 3 in the bodily waste of the subject K at Step S102 (NO at Step S103), the user determines that the pretest capsule 1 does not reach the large intestine of the subject K within the predetermined time (Step S107). Based on a result of this determination, the user determines that a stenosis part exists within the lumen of the subject K, and determines that when the subject K orally ingests the capsule endoscope, the capsule endoscope stagnates at the stenosis part and does not easily reach the large intestine within the predetermined time (that is, there is a problem in the passability of the capsule endoscope through the lumen of the subject K).

Next, the user prompts the subject K having the problem of the passability of the capsule endoscope through the lumen to orally ingest a dissolving liquid of the pretest capsule 1 (Step S108), thereby removing the pretest capsule 1 stagnated at the stenosis part of the subject K. When the pretest-capsule 1 does not reach the large intestine of the subject K within the predetermined time, the pretest capsule 1 stagnates at the stenosis part within the lumen. Therefore, the user prompts the subject K to ingest the dissolving liquid of the pretest capsule 1 at Step S108, thereby collapsing the outer case 2 of the pretest capsule 1 remaining within the lumen of the subject K and discharging the pretest capsule 1 to the outside of the body of the subject K. For the dissolving liquid of the pretest capsule 1, there can be listed a substance within the large intestine (a bacterium within large intestine, an enzyme generated by a bacterium within the large intestine or the like) that can dissolve the outer case 2 (a specific enteric substance), and a liquid (a liquid containing an enzyme generated by the bacterium within the large intestine or the like) that simulates a substance within the large intestine. More specifically, when the outer case 2 (a specific enteric substance) is chitosan, this chitosan is also dissolved by an organic-acid aqueous solution. Therefore, it suffices that the subject K ingests a liquid containing acetic solution (an example of an organic-acid aqueous solution) as a dissolving liquid.

Thereafter, the user determines not to perform the capsule endoscope examination on the subject K who has a problem in the passability of the capsule endoscope through the lumen as described above (Step S109), and completes the process of checking the passability of the capsule endoscope through the lumen of the subject K.

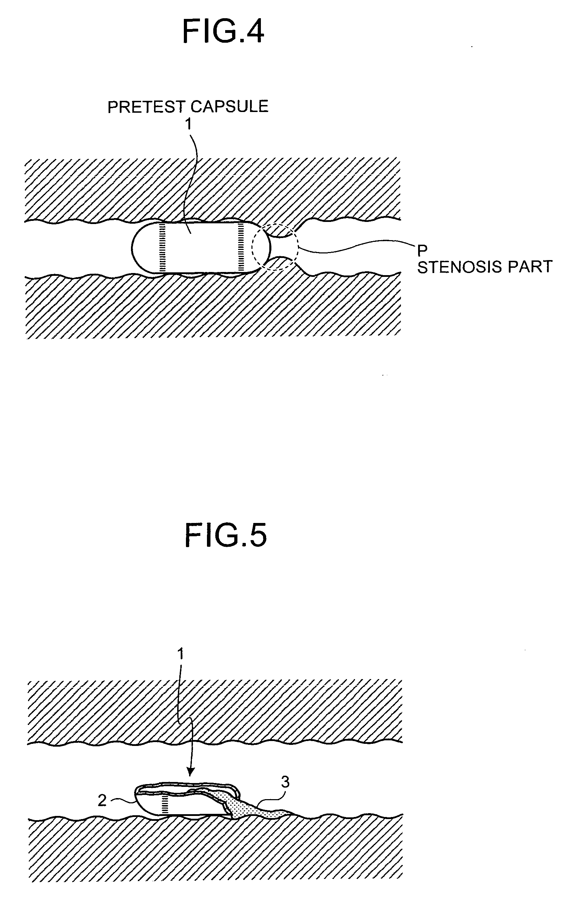

Stagnation of the pretest capsule 1 orally ingested by the subject K is described next. FIG. 4 is a schematic diagram for exemplifying a state that the pretest capsule 1 stagnates at the stenosis part within the lumen of the subject K. After the subject K orally ingests the pretest capsule 1, the pretest capsule 1 advances within the lumen of the subject K by a peristaltic movement or the like. When a stenosis part exists within the lumen of the subject K, the pretest capsule 1 stagnates at the stenosis part.

Specifically, as shown in FIG. 4, when a stenosis part P smaller than an external diameter of the outer case 2 exists within the lumen (small intestine, for example) of the subject K, the advancement of the pretest capsule 1 is blocked by the stenosis part P of the small intestine and the pretest capsule 1 stagnates there. In this case, the pretest capsule 1 cannot reach the organ (large intestine) beyond the stenosis part P of the small intestine.

The stenosis part P that blocks the advancement of the pretest capsule 1 similarly blocks the advancement of the capsule medical device having an external diameter equal to that of the pretest capsule 1. That is, this capsule medical device stagnates at the stenosis part P and cannot reach the large intestine as a reach target region. Consequently, the subject K having the stenosis part P within the lumen is determined to have a problem in the passability of the capsule medical device through the lumen.

Meanwhile, the pretest capsule 1 stagnated at the stenosis part P can be removed by the dissolving liquid orally ingested by the subject K at Step S108 described above. Specifically, the outer case 2 of the pretest capsule 1 is dissolved by this dissolving liquid, and is cracked (or collapsed) in a state sufficiently smaller than the stenosis part P. Thereafter, the rest of the collapsed outer case 2 easily passes through the stenosis part P by a peristaltic movement or the like, and is excreted from the body of the subject K together with his bodily waste after a lapse of sufficient time. The pigment 3 contained in the outer case 2 is similarly excreted from the body of the subject K together with the rest of the outer case 2.

Discharge of the pigment 3 from the pretest capsule 1 reaching the inside of the target organ of the subject K is described next. FIG. 5 is a schematic diagram for exemplifying a state that the pigment 3 is discharged from the pretest capsule 1 having reached the target organ of the subject K. After the subject K orally ingests the pretest capsule 1, the pretest capsule 1 advances within the lumen of the subject K by a peristaltic movement or the like. When there is no stenosis part within the lumen of the subject K, the pretest capsule 1 safely passes through the inside of the lumen of the subject K, and reaches the large intestine as the reach target region of the capsule medical device. The pretest capsule 1 reaching the large intestine discharges the pigment 3 along the dissolution of the outer case.

Specifically, as shown in FIG. 5, when the pretest capsule 1 reaches the large intestine, the outer case 2 of the pretest capsule 1 is dissolved by a specific substance within the large intestine (a bacterium within the large intestine or an enzyme generated by the bacterium within the large intestine), and is collapsed after a lapse of sufficient time. On the other hand, the pigment 3 contained in the outer case 2 is discharged to the inside of the large intestine along the dissolution of the outer case 2. The pigment 3 discharged to the inside of the large intestine is excreted from the body of the subject K together with his bodily waste. In this case, the pigment 3 is excreted from the body in the state of coloring the bodily waste of the subject K, thereby notifying the outside (the user such as a doctor or nurse or the subject K) that the pretest capsule 1 has reached the large intestine (the reach target region of the capsule medical device). The user such as a doctor or nurse visually confirms the pigment 3 mixed in the bodily waste (that is, the bodily waste colored by the pigment 3), thereby determining that the pretest capsule 1 has reached the large intestine as the reach target region of the capsule medical device. Based on a result of this determination, the user can determine that the capsule medical device can safely pass through the inside of the lumen of the subject K and reach the large intestine (that is, there is no problem in the passability of the capsule medical device through the lumen of the subject K).

As described above, in the first embodiment according to the present invention, an outer structure having an external diameter substantially equal to that of a capsule medical device inserted into the body of a subject is formed by using a member dissolved by a specific substance existing within a target organ. A visually recognizable identifier such as a pigment is filled into the outer structure. This identifier is discharged from the outer structure along the dissolution of the outer structure within the target organ as a reach target region of the capsule medical device. The subject orally ingests the outer structure containing this identifier. Thereafter, a bodily waste excreted from the subject during a period until when a predetermined time elapses is visually recognized. Based on a result of this visual check as to whether the identifier is included in the bodily waste of the subject (for example, whether the bodily waste of the subject is colored by the pigment), it is determined whether the capsule medical device reaches the target organ within a predetermined time after the subject orally ingests the capsule medical device. Therefore, it is easy to provide a lumen passability checking device and a lumen passability checking method capable of easily checking whether the capsule medical device reaches the target organ within the predetermined time by safely passing through the inside of the lumen (that is, the passability of the capsule medical device through the lumen until when the device reaches the target organ within the subject), based on a result of the visual check of the bodily waste of the subject, without checking presence of the outer structure or the identifier by using an apparatus outside the subject such as an RF-ID communication apparatus or an X-ray apparatus.

Because the outer structure is formed by using a specific enteric member that is dissolved by a specific substance within the large intestine, the outer structure maintains an external diameter equal to that of the capsule medical device during a period from when the subject orally ingests the outer structure until when the outer structure reaches the large intestine. Further, the outer structure can maintain a state of containing the identifier. When the outer structure reaches the inside of the large intestine of the subject, the outer structure is dissolved by a specific substance within the large intestine, and the inside identifier can be discharged to the inside of the large intestine along this dissolution. As a result, it is easy to provide the lumen passability checking device and the lumen passability checking method capable of easily checking whether the capsule medical device reaches the large intestine within the predetermined time by safely passing through the inside of the lumen (that is, the passability of the capsule medical device through the lumen until when the device reaches the large intestine of the subject).

Further, even when the outer structure stagnates at the stenosis part within the lumen of the subject, this outer structure can be easily collapsed by a substance within the large intestine or when the subject ingests a dissolving liquid such as a liquid simulating the substance within the large intestine. As a result, this outer structure can be easily excreted from the body of the subject.

Because an external shape of the outer structure is formed in a capsule shape similar to that of the capsule medical device, the passability of the capsule medical device through the lumen of the subject can be checked in a state similar to a state that the capsule medical device is actually inserted into the body of the subject.

### (First modification of first embodiment)

A first modification of the first embodiment according to the present invention is described next. A pretest capsule (an example of a lumen passability checking device) according to the first modification of the first embodiment includes an outer case of a two-layer configuration including an internal dissoluble unit formed by a specific enteric member described above and an external dissoluble unit formed by a general enteric member.

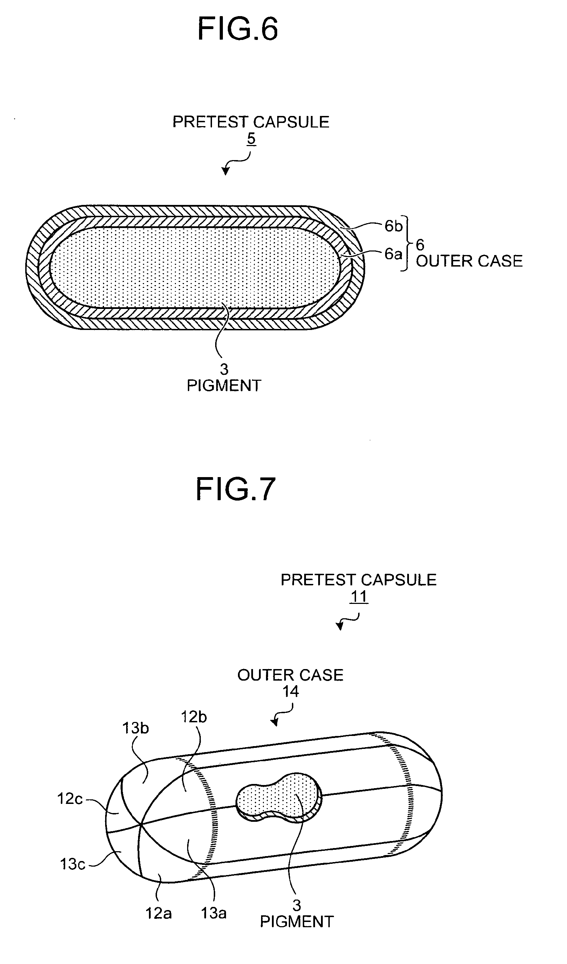

FIG. 6 is a vertical cross-sectional schematic diagram of a configuration example of the pretest capsule according to the first modification of the first embodiment of the present invention. As shown in FIG. 6, a pretest capsule 5 according to the first modification of the first embodiment has an outer case 6 of a two-layer configuration in place of the outer case 2 of the pretest capsule 1 according to the first embodiment. Other configurations are the same as those of the first embodiment, and like constituent elements are denoted by like reference numerals.

The outer case 6 is an outer structure of a two-layer configuration having an internal dissoluble unit 6a formed in a capsule shape and an external dissoluble unit 6b formed in a capsule shape. The internal dissoluble unit 6a is a structure of a capsule shape formed by a specific enteric member such as chitosan, and contains the pigment 3. The internal dissoluble unit 6a is dissolved by a specific substance within the large intestine, and discharges the inside pigment 3 along this dissolution. The internal dissoluble unit 6a can be formed by only a specific enteric member or can be the one having a specific enteric member coated on an external wall of the structure formed by a general enteric member such as gelatin.

The external dissoluble unit 6b is a structure formed by a general enteric member such as gelatin, and is arranged at the outside of the internal dissoluble unit 6a in a capsule shape substantially identical to that of the internal dissoluble unit 6a. The external dissoluble unit 6b protects the internal dissoluble unit 6a by covering the whole of the internal dissoluble unit 6a. The external dissoluble unit 6b has a thickness to the extent not to expose the internal dissoluble unit 6a before passing through the inside of the small intestine.

The outer case 6 of the two-layer configuration functions as an outer structure of the pretest capsule 5, and is formed in a size that can be inserted into the body of the subject. Specifically, the outer case 6 has an external diameter substantially identical to that of a capsule medical device inserted into the body of the subject. Preferably, the outer case 6 is formed in an external shape (a capsule shape) similar to that of the capsule medical device. Even when an external pressure is applied to the outer case 6 by a peristaltic movement of the organ, the outer case 6 maintains the external diameter substantially equal to that of the capsule medical device (further, an external shape similar to that of the capsule medical device).

The subject orally ingests the pretest capsule 5 having this outer case 6 in a similar manner to that of the pretest capsule 1 according to the first embodiment. Thereafter, when there is no stenosis part within the lumen of the subject, the pretest capsule 5 safely passes through the inside of the lumen, and reaches the large intestine as the reach target region of the capsule medical device.
When a stenosis part exists within the lumen of the subject, the pretest capsule 5 stagnates at this stenosis part (that is, the pretest capsule 5 does not reach the large intestine within a predetermined time). When the pretest capsule 5 reaches the large intestine, both the external dissoluble unit 6b and the internal dissoluble unit 6a of the outer case 6 are dissolved by a specific substance within the large intestine, and the pigment 3 contained in the internal dissoluble unit 6a is discharged from the outer case 6. That is, the user such as a doctor or nurse performs a process procedure along Steps S101 to S109 described above (see FIG. 3) by using the pretest capsule 5. With this arrangement the user can easily check the passability of the capsule medical device through the lumen of the subject in a similar manner to that of the first embodiment. Further, the rest of the outer case 6 and the pigment 3 remaining within the body of the subject can be removed.

At the end of the small intestine near the large intestine of the subject, there is a position into which a specific substance existing within the large intestine may flow. When the internal dissoluble unit 6a not covered (not protected) by the external dissoluble unit 6b reaches the end of the small intestine, for example, there is a possibility that the internal dissoluble unit 6a is dissolved by being in contact, for a predetermined time or more, with a specific substance originated from the large intestine (hereinafter, referred to as "substance of large-intestine origin") that flows into the end of the small intestine, and the external dissoluble unit 6b is collapsed before advancing from the end of the small intestine to the large intestine.

On the other hand, even when the outer case 6 of the two-layer configuration protecting the internal dissoluble unit 6a by the external dissoluble unit 6b reaches the end of the small intestine where the substance of large-intestine origin exists, the external dissoluble unit 6b can block a contact between the substance of large-intestine origin and the internal dissoluble unit 6a during a predetermined time. As a result, the internal dissoluble unit 6a can reach the inside of the large intestine from the end of the small intestine, before the internal dissoluble unit 6a is dissolved by the substance of large-intestine origin. The outer case 6 can securely maintain the external shape (the capsule shape) of the internal dissoluble unit 6a containing the pigment 3 during the period until when the outer case 6 reaches the large intestine after passing through the inside of the lumen of the subject. Consequently, it is possible to securely test the passability of the capsule medical device through the lumen over the whole region of the small intestine including the end of the small intestine in which the substance of large-intestine origin exists.

As explained above, according to the first modification of the first embodiment of the present invention, the pretest capsule has an outer structure of the two-layer configuration including the internal dissoluble unit formed by a specific enteric member and the external dissoluble unit formed by a general enteric member. Further, the pretest capsule includes a visually checkable pigment within the internal dissoluble unit, and has other parts configured in a similar manner to that of the first embodiment. The pretest capsule can securely maintain the external shape of the internal dissoluble unit containing the identifier during a period until when the outer structure reaches the large intestine after passing through the inside of the lumen of the subject. Consequently, there is an operational effect similar to that of the first embodiment, and it is possible to securely test the passability of the capsule medical device through the lumen over the whole region of the small intestine including the end of the small intestine in which the substance of large-intestine origin exists.

### (Second modification of first embodiment)

A second modification of the first embodiment of the present invention is described next. A pretest capsule (an example of a lumen passability checking device) according to the second modification of the first embodiment includes an outer case formed by connecting between a large-intestine dissoluble unit that can be dissolved by a specific substance within the large intestine and a limonene dissoluble unit that can be dissolved by a limonene liquid. The limonene dissoluble unit can be any specific dissoluble unit made of a specific dissoluble material that is not dissolved by a specific substance within the large intestine as well as limonene and that has biocompatibility of being dissolved by other specific substance (liquid).

FIG. 7 is a schematic diagram of a configuration example of the pretest capsule according to the second modification of the first embodiment of the present invention. FIG. 7 depicts the pretest capsule in a state that a part of an outer structure is broken to explain an internal configuration of the pretest capsule according to the second modification of the first embodiment. As shown in FIG. 7, a pretest capsule 11 according to the second modification of the first embodiment has an outer case 14 in place of the outer case 2 of the pretest capsule 1 according to the first embodiment. Other configurations are the same as those of the first embodiment, and like constituent elements are denoted by like reference numerals.

The outer case 14 is a structure having a capsule shape formed by alternately connecting in a circumferential direction large-intestine dissoluble units 12a to 12c that can be dissolved by a specific substance within the large intestine and limonene dissoluble units 13a to 13c that can be dissolved by a limonene liquid. The outer case 14 has the limonene dissoluble unit 13a between the large-intestine dissoluble units 12a and 12b, has the limonene dissoluble unit 13b between the large-intestine dissoluble units 12b and 12c, and has the limonene dissoluble unit 13c between the large-intestine dissoluble units 12c and 12a.

The large-intestine dissoluble units 12a to 12c are formed by using a specific enteric member that can be dissolved by a specific substance within the large intestine. The large-intestine dissoluble units 12a to 12c can be formed by only a specific enteric member, or can be the one having a specific enteric member coated on an external wall of the structure formed by a general enteric member such as gelatin. On the other hand, the limonene dissoluble units 13a to 13c are formed by polymers having a molecular structure similar to that of limonene, and are dissolved by a limonene liquid.

The outer case 14 formed by the large-intestine dissoluble units 12a to 12c and the limonene dissoluble units 13a to 13c functions as an outer structure of the pretest capsule 11, and contains the pigment 3. The outer case 14 has an external diameter substantially identical to that of the capsule medical device inserted into the body of the subject. Preferably, the outer case 14 has an external shape similar to that of the capsule medical device. Even when an external pressure is applied to the outer case 14 by a peristaltic movement of the organ, the outer case 14 maintains an external diameter substantially equal to that of the capsule medical device (further, an external shape similar to that of the capsule medical device), and is collapsed in the existence of a limonene liquid or a specific substance within the large intestine.

The number of each of the large-intestine dissoluble units and the limonene dissoluble units forming the outer case 14 is not limited to three. As far as the outer case 14 can be shrink-deformed or can be collapsed, when either the large-intestine dissoluble unit or the limonene dissoluble unit is dissolved, each number can be one or more. While the outer case 14 is formed by alternately connecting the large-intestine dissoluble units and the limonene dissoluble units in the circumferential direction, the outer case 14 can be formed by alternately connecting the large-intestine dissoluble units and the limonene dissoluble units in a longitudinal direction of the capsule shape.

The subject orally ingests the pretest capsule 11 having this outer case 14 in a similar manner to that of the pretest capsule 1 according to the first embodiment. When there is no stenosis part within the lumen of the subject, the pretest capsule 11 safely passes through the inside of the lumen, and reaches the large intestine as the reach target region of the capsule medical device. The pretest capsule 11 reaching the large intestine causes the outer case 14 to be collapsed by the dissolution of the large-intestine dissoluble units 12a to 12c by a specific substance within the large intestine, and discharges the pigment 3 contained in the outer case 14.

On the other hand, when a stenosis part exists within the lumen of the subject, the pretest capsule 11 stagnates at this stenosis part (that is, the pretest capsule 11 does not reach the large intestine within a predetermined time). In this case, the user such as a doctor or nurse prompts the subject to orally ingest a limonene liquid as a dissolving liquid of the pretest capsule. The limonene dissoluble units 13a to 13c of the pretest capsule 11 stagnated at this stenosis part are dissolved by the limonene liquid orally ingested by the subject. When the limonene dissoluble units 13a to 13c are dissolved, the pretest capsule 11 (specifically, the outer case 14) stagnated at the stenosis part is collapsed. Thereafter, the rest of the collapsed outer case 14 (that is, the large-intestine dissoluble units 12a to 12c) reaches the large intestine after easily passing through the stenosis part, and is dissolved by a specific substance within the large intestine.

That is, the user such as a doctor or nurse performs the process procedure along Steps S101 to S109 described above (see FIG. 3) by using the pretest capsule 11. With this arrangement the user can easily check the passability of the capsule medical device through the lumen of the subject in a similar manner to that of the first embodiment. Further, the rest of the outer case 14 and the pigment 3 remaining within the body of the subject can be removed. In the second modification of the first embodiment, at Step S108 described above, the subject ingests the limonene liquid as an example of the dissolving liquid of the pretest capsule.

As explained above, in the second modification of the first embodiment of the present invention, the pretest capsule has an outer structure formed by connecting the large-intestine dissoluble unit that is dissolved by a specific substance within the large intestine and the limonene dissoluble unit that is dissolved by the limonene liquid. Further, the pretest capsule includes a visually checkable identifier such as a pigment within the outer structure, and has other parts configured in a similar manner to that of the first embodiment. Therefore, the specific substance within the large intestine or the limonene liquid orally ingested by the subject can collapse the outer structure within the lumen. Consequently, there is an operational effect similar to that of the first embodiment, and a passability test of the capsule medical device through the lumen can be easily performed.

### (Second embodiment)

A second embodiment of the present invention is described next. In the first embodiment, the outer structure is collapsed by bringing the outer structure into contact with a specific substance within large intestine or a dissolving liquid orally ingested by the subject. On the other hand, in the second embodiment, an outer structure is shrink-deformed or is collapsed by bringing the outer structure into contact with a specific substance within the large intestine or by keeping the outer structure in contact with body fluid within the lumen for a predetermined time or more.

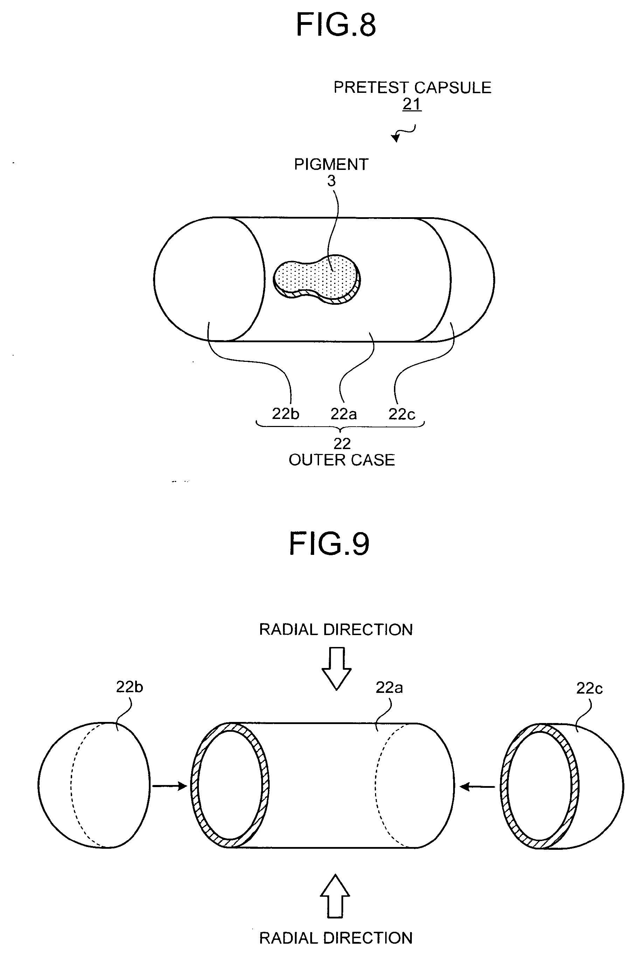

FIG. 8 is a schematic diagram of a configuration example of a pretest capsule according to the second embodiment of the present invention. FIG. 9 is a schematic diagram of a state that the outer structure of the pretest capsule is broken into a body unit and end units. FIG. 8 depicts the pretest capsule in a state that a part of the outer structure is broken to explain an internal configuration of the pretest capsule (an example of a lumen passability checking device) according to the second embodiment. As shown in FIG. 8, a pretest capsule 21 according to the second embodiment has an outer case 22 in place of the outer case 2 of the pretest capsule 1 according to the first embodiment. Other configurations are the same as those of the first embodiment, and like constituent elements are denoted by like reference numerals.

The outer case 22 functions as an outer structure of the pretest capsule 21, and contains the pigment 3. Further, the outer case 22 has an external diameter substantially identical to that of the capsule medical device inserted into the body of the subject. Preferably, the outer case 22 is formed to have an external shape similar to that of the capsule medical device.
Specifically, the outer case 22 is formed by connecting domed lids 22b and 22c to both openings of a body unit 22a of a cylindrical structure as shown in FIG. 9. Even when an external pressure is applied to the outer case 22 by a peristaltic movement of the organ, the outer case 22 maintains an external diameter substantially equal to that of the capsule medical device (further, an external shape similar to that of the capsule medical device).

The body unit 22a is a cylindrical structure formed by using a specific enteric member that is dissolved by a specific substance within the large intestine. The body unit 22a forms an external diameter of the outer case 22 (that is, an external diameter substantially equal to that of the capsule medical device), and is easily shrink-deformed by an external pressure from a radial direction (see FIG. 9). On the other hand, the body unit 22a maintains its external diameter even when an external pressure is applied to the body unit 22a by a peristaltic movement from a radial direction, when the lids 22b and 22c are fitted to both opening ends.

The body unit 22a can be formed by only a specific enteric member, or can be the one having a specific enteric member coated on an external wall of the structure formed by a general enteric member such as gelatin.

The lids 22b and 22c are domed structures formed by a member (for example, gelatin or lactose) that is dissolved when this member is in contact for a predetermined time or more with body fluid existing within the lumen of the subject. The lids 22b and 22c are fitted to both opening ends of the body unit 22a to close both opening ends of the body unit 22a, and increase strength of the body unit 22a. Specifically, even when an external force (an external pressure by a peristaltic movement, for example) is applied from a radial direction to the body unit 22a, the lids 22b and 22c fitted to both opening ends of the body unit 22a prevent a shrink deformation of the body unit 22a, and maintain the external diameter of the body unit 22a. When the lids 22b and 22c are provided by pressure forming a general enteric substance such as gelatin or lactose to a predetermined thickness, the lids 22b and 22c maintain their shapes until when the lids 22b and 22c are in contact with the body fluid of the subject for a predetermined time or more.

As described above, even when an external force in a radial direction is applied by a peristaltic movement or the like, the outer case 22 formed by the body unit 22a and the lids 22b and 22c maintains an external diameter substantially equal to that of the capsule medical device (and further, an external shape similar to that of the capsule medical device). On the other hand, when the outer case 22 is positioned (stagnated) within the lumen for a predetermined time or more by a stenosis part or the like within the lumen, for example, the outer case 22 is in contact with the body fluid within the lumen for a predetermined time or more, thereby losing the lids 22b and 22c. In this way, the rest of the outer case 22 having lost the lids 22b and 22c (that is, the body unit 22a) is easily shrink-deformed or collapsed by an external force in a radial direction by a peristaltic movement or the like.

The time necessary for the lids 22b and 22c of the outer case 22 to be dissolved by the body fluid within the lumen after the lids 22b and 22c are brought into contact with the body fluid within the lumen is sufficiently long as compared with a time necessary for the capsule medical device to reach the reach target region (for example, large intestine) by passing through the inside of the lumen after the device is orally ingested by the subject.

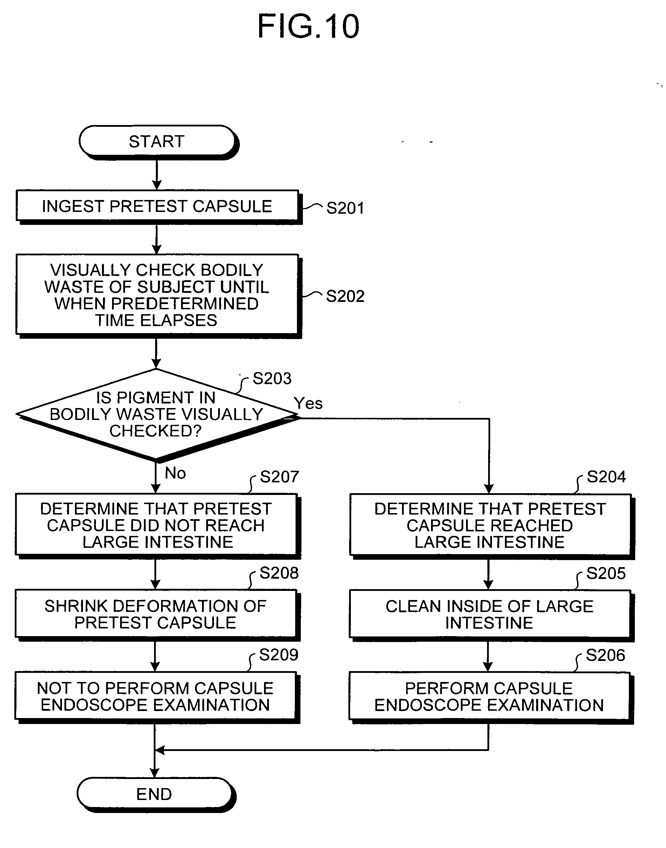

A method of checking a passability of the capsule medical device through the lumen by using the pretest capsule 21 according to the second embodiment of the present invention is described next. FIG. 10 is a flowchart for exemplifying the lumen passability checking method according to the second embodiment of the present invention. The lumen passability checking method according to the second embodiment of the present invention is described below by exemplifying a case of checking beforehand a passability of the capsule endoscope through the lumen of the subject K before performing a capsule endoscope examination.

In FIG. 10, in a similar manner to that at Steps S101 and S102 described above, a user such as a doctor or nurse prompts the subject K to ingest the pretest capsule 21 having an external diameter equal to that of the capsule endoscope to be inserted into the body of the subject K (Step S201). The user visually checks a bodily waste excreted from the subject K during a period from when the subject K ingests the pretest capsule 21 until when a predetermined time (20 hours, for example) elapses (Step S202).

After the subject K ingests the pretest capsule 21, the pretest capsule 21 advances within the lumen of the subject K by a peristaltic movement or the like. When there is no stenosis part within the lumen of the subject K, the pretest capsule 21 reaches the large intestine as the reach target region of the capsule endoscope. The pretest capsule 21 reaching the large intestine is collapsed by the dissolution of the body unit 22a by a specific substance within the large intestine, and discharges the pigment 3 contained in the outer case 22. On the other hand, when a stenosis part exists within the lumen of the subject K, the pretest capsule 21 stagnates at this stenosis part. At Step S202, the user checks whether the pigment 3 can be visually checked in the bodily waste excreted from the subject K by the time when a predetermined time elapses after the subject K orally ingests the pretest capsule 21 (that is, whether the bodily waste of the subject K is colored by the pigment 3).

At Step S202, the user can visually check the bodily waste excreted from the subject K after a lapse of a predetermined time since the subject K ingests the pretest capsule 21, or can visually check plural bodily wastes excreted from the subject K sequentially at plural times during a period until when the predetermined time elapses.

When the user visually checks that the bodily waste of the subject K is colored by the pigment 3, that is, when the user visually checks the pigment 3 in the bodily waste of the subject K at Step S202 (YES at Step S203), the user determines, in a similar manner to that at Step S104 described above, that the pretest capsule 21 reaches the large intestine of the subject K within the predetermined time (Step S204). Based on a result of this determination, the user determines that when the subject K orally ingests the capsule endoscope, this capsule endoscope reaches the large intestine within a predetermined time by safely passing through the inside of the lumen (that is, there is no problem in the lumen passability of the capsule endoscope regarding the subject K).

Thereafter, in a similar manner to that at Steps S105 and S106 described above, the user cleans the inside of the large intestine of the subject K who has no problem in the passability of the capsule endoscope through the lumen (Step S205), and performs the capsule endoscope examination on the subject K whose inside of the large intestine is cleaned (Step S206). Consequently, a capsule-endoscope lumen-passability checking process to the subject K is completed.

On the other hand, when the user visually confirms at Step S202 that the bodily waste of the subject K is not colored by the pigment 3, that is, when the user does not visually find the pigment 3 in the bodily waste of the subject K at Step S202 (NO at Step S203), the user determines, in a similar manner to that at Step S107 described above, that the pretest capsule 21 does not reach the large intestine of the subject K within the predetermined time (Step S207). Based on a result of this determination, the user determines that a stenosis part exists within the lumen of the subject K, and determines that when the subject K orally ingests the capsule endoscope, the capsule endoscope stagnates at the stenosis part and does not easily reach the large intestine within the predetermined time (that is, there is a problem in the passability of the capsule endoscope through the lumen of the subject K).

When the user determines that there is a problem in the passability of the capsule endoscope through the lumen of the subject K, the user has the pretest capsule 21 stagnated at the stenosis part within the lumen of the subject K shrink-deformed (Step S208). At Step S208, the user causes the pretest capsule 21 stagnated at the stenosis part to be in contact with the body fluid within the lumen for a predetermined time or more (that is, has the lids 22b and 22c dissolved by the body fluid within the lumen), thereby shrink-deforming the pretest capsule 21. The rest of the shrink-deformed pretest capsule 21 easily passes through the stenosis part within the lumen, and is excreted from the body of the subject K.

Thereafter, the user determines not to perform the capsule endoscope examination on the subject K who has a problem in the passability of the capsule endoscope through the lumen as described above (Step S209), and completes the process of checking a passability of the capsule endoscope through the lumen of the subject K.

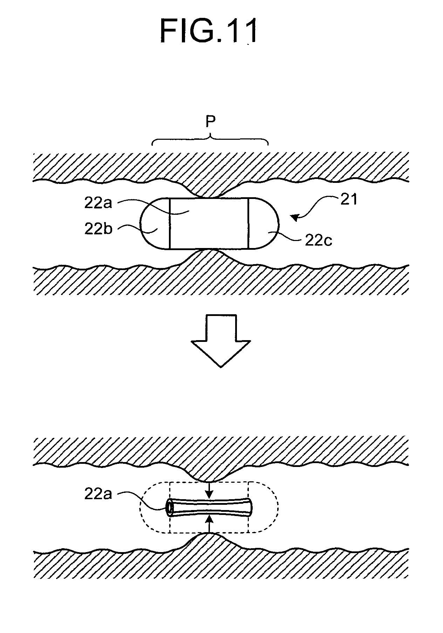

Shrink deformation of the pretest capsule 21 stagnated at the stenosis part within the lumen is described next. FIG. 11 is a schematic diagram for explaining shrink deformation of the pretest capsule 21 having stagnated at the stenosis part within the lumen. As shown in FIG. 11, when the stenosis part P smaller than an external diameter of the outer case 22 exists within the lumen (for example, small intestine) of the subject K, the advancement of the pretest capsule 21 is blocked by the stenosis part P of the small intestine, and stagnates there. The pretest capsule 21 stagnated at the stenosis part P cannot reach the subsequent organ (that is, the large intestine).

The pretest capsule 21 continues to be stagnated at the stenosis part P, and becomes in a state of being in contact with the body fluid within the small intestine for a predetermined time or more. In this case, the lids 22b and 22c of the outer case 22 are dissolved by the body fluid within the small intestine. In this way, the pretest capsule 21 loses the lids 22b and 22c. The rest of the outer case 22 after the lids 22b and 22c are dissolved, that is, the body unit 22a, is easily shrink-deformed or collapsed by an external force in a radial direction by a peristaltic movement or the like, as shown in FIG. 11. The shrink-deformed or collapsed body unit 22a easily passes through the stenosis part P, and is excreted from the body of the subject K.

On the other hand, when there is no stenosis part within the lumen of the subject K, the pretest capsule 21 safely passes through the inside of the lumen and reaches the large intestine as the reach target region of the capsule medical device within a predetermined time, in a similar manner to that of the pretest capsule 1 according to the first embodiment. The pretest capsule 21 reaching the large intestine causes the outer case 22 to be collapsed by the dissolution of the body unit 22a based on a specific substance within the large intestine, thereby discharging the pigment 3 contained in the outer case 22.

As described above, in the second embodiment of the present invention, the pretest capsule has an outer structure formed by connecting lids dissolved when contacted, during a predetermined time or more, to the body fluid within the lumen, at both opening ends of the body unit of a cylindrical configuration dissolved by a specific substance within the large intestine. The outer structure includes therein an identifier that can be visually checked such as the pigment, and other configurations are substantially identical to those of the first embodiment. Therefore, even when the outer structure stagnates at the stenosis part within the lumen of the subject, the outer structure can be easily shrink-deformed or collapsed by contacting the outer structure to the body fluid within the lumen for a predetermined time or more. Accordingly, the outer structure stagnated within the lumen of the subject for the predetermined time or more can be easily excreted from the body without causing the subject to orally ingest a dissolving liquid of the outer structure. Consequently, there is an operational effect similar to that of the first embodiment, and the subject is not required to orally ingest the dissolving liquid of the outer structure.

### (Third embodiment)

A third embodiment of the present invention is described next. In the first embodiment described above, a type of identifier (the pigment 3 having one color, for example) is filled into an internal space of the outer structure. By visually checking whether the identifier is contained in the bodily waste, the passability of a capsule medical device through the lumen of the subject is checked. On the other hand, in the third embodiment, two types of identifiers (pigments of two colors, for example) are filled into internal spaces of the outer structure. By visually checking how many types of identifiers are contained in the bodily waste of the subject, a passability of a capsule medical device through the lumen of the subject is checked.

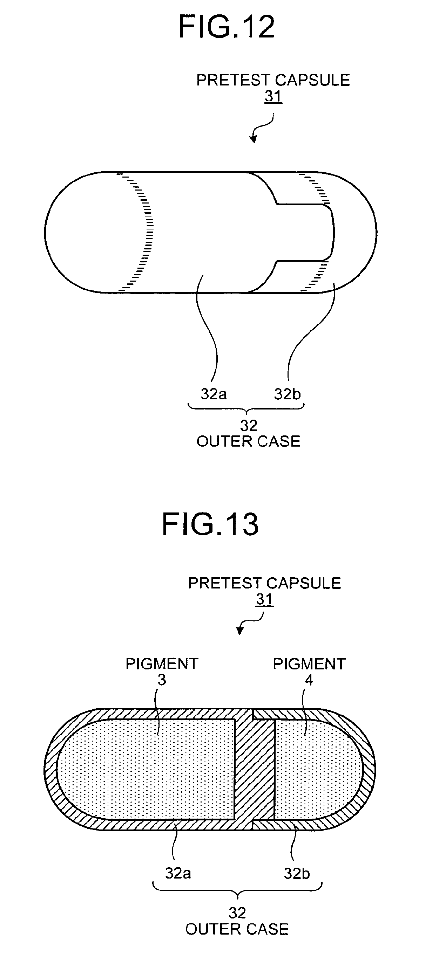

FIG. 12 is a schematic diagram of a configuration example of a pretest capsule according to the third embodiment of the present invention. FIG. 13 is a cross-sectional schematic diagram for exemplifying a vertical cross-sectional configuration of the pretest capsule according to the third embodiment of the present invention. As shown in FIGS. 12 and 13, a pretest capsule 31 (an example of a lumen passability checking device) according to the third embodiment has an outer case 32 in place of the outer case 2 of the pretest capsule 1 according to the first embodiment. The outer case 32 further contains a pigment 4. In this case, the outer case 32 separately contains two types of pigments separated by colors. Other configurations are the same as those of the first embodiment, and like constituent elements are denoted by like reference numerals.

The outer case 32 functions as an outer structure of the pretest capsule 31, and contains two types of the pigments 3 and 4 separated by colors. The outer case 32 has an external diameter substantially identical to that of the capsule medical device inserted into the body of the subject. Preferably, the outer case 32 has an external shape similar to that of the capsule medical device. Specifically, as shown in FIGS. 12 and 13, the outer case 32 is formed by connecting (by engaging, for example) a small-intestine dissoluble unit 32b that can be dissolved by bile as strong alkali, to one end of a large-intestine dissoluble unit 32a that can be dissolved by a specific substance within the large intestine. The outer case 32 maintains an external diameter substantially equal to that of the capsule medical device (and further an external diameter similar to that of the capsule medical device) even when an external pressure is applied to the outer case 32 by a peristaltic movement of the organ.

The large-intestine dissoluble unit 32a is a structure of substantially a capsule shape formed by a specific enteric member such as chitosan, and contains the pigment 3. The large-intestine dissoluble unit 32a is dissolved by a specific substance within the large intestine, and discharges the inside pigment 3 along this dissolution. The large-intestine dissoluble unit 32a also forms substantially the whole of the outer case 32. That is, the large-intestine dissoluble unit 32a forms an external diameter of the outer case 32. Even when the small-intestine dissoluble unit 32b connected to one of the large-intestine dissoluble unit 32a is dissolved, the large-intestine dissoluble unit 32a maintains this external diameter. The large-intestine dissoluble unit 32a can be formed by only a specific enteric member, or can be the one having a specific enteric member coated on an external wall of the structure formed by a general enteric member such as gelatin.

The small-intestine dissoluble unit 32b is a structure formed by a general enteric member such as gelatin, and is connected (fitted) to one end of the large-intestine dissoluble unit 32a. Specifically, the small-intestine dissoluble unit 32b has a vertical cross section of a substantially U shape, and forms one end (a dome shape) of a capsule shape of the outer case 32. The small-intestine dissoluble unit 32b forms a closed space encircled by an internal wall of the small-intestine dissoluble unit 32b and one end of the large-intestine dissoluble unit 32a, and contains the pigment 4 within this closed space. When the pretest capsule 31 reaches the inside of the small intestine, the small-intestine dissoluble unit 32b is dissolved by the bile within the small intestine, and discharges the inside pigment 4 along the dissolution. The small-intestine dissoluble unit 32b is also dissolved by a substance within the large intestine.

The pigment 4 is an edible dye safe for a living body, and has a color different from that of the pigment 3. As shown in FIG. 13, the pigment 4 is filled into a closed space encircled by the external wall of one end of the large-intestine dissoluble unit 32a and the internal wall of the small-intestine dissoluble unit 32b, and is discharged to the inside of the small intestine from the small-intestine dissoluble unit 32b along the dissolution of the small-intestine dissoluble unit 32b by the bile within the small intestine. Thereafter, the pigment 4 discharged from the small-intestine dissoluble unit 32b is excreted from the body together with the bodily waste of the subject. In this case, the pigment 4 colors the bodily waste of the subject in a color different from that of the pigment 3, thereby notifying the outside (a doctor or nurse) that the pretest capsule 31 reaches the small intestine. The pigment 4 functions as an identifier that can be visually identified to check whether the pretest capsule 31 has reached the small intestine, that is, whether the capsule medical device inserted into the body of the subject reaches the small intestine.

The pigment 4 can be an edible dye having a color different from that of the pigment 3. Preferably, the pigment 4 is an edible dye having a color other than that having a possibility of being included in the bodily waste of the subject (such as red, white, black, green, and yellow), that is, a color such as blue or aqua that is not usually included in the bodily waste. Further, a state of the pigment 4 can be a powder (a particle shape) or a liquid shape (for example, the pigment dissolved in a liquid such as water or a normal saline solution).

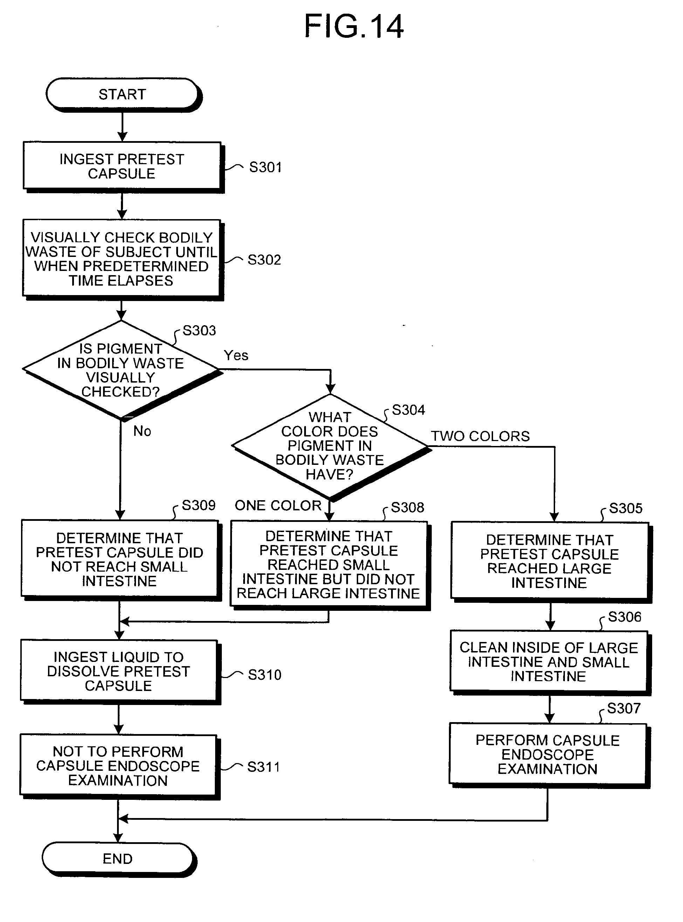

A method of checking a passability of the capsule medical device through the lumen by using the pretest capsule 31 according to the third embodiment of the present invention is described next. FIG. 14 is a flowchart for exemplifying the lumen passability checking method according to the third embodiment of the present invention. The lumen passability checking method according to the third embodiment of the present invention is described below by exemplifying a case of checking beforehand a passability of the capsule endoscope through the lumen of the subject K before performing a capsule endoscope examination.

In FIG. 14, in a similar manner to that at Step S101 described above, a user such as a doctor or nurse prompts the subject K to ingest the pretest capsule 31 having an external diameter equal to that of the capsule endoscope to be inserted into the body of the subject K (Step S301). The user visually checks a bodily waste excreted from the subject K during a period from when the subject K ingests the pretest capsule 31 until when a predetermined time (20 hours, for example) elapses (Step S302). At Step S302, the user sequentially visually checks plural bodily wastes excreted from the subject K at plural times during a period until when the predetermined time elapses after the subject K ingests the pretest capsule 31.

After the subject K ingests the pretest capsule 31, the pretest capsule 31 advances within the lumen of the subject K by a peristaltic movement or the like. When there is no stenosis part within the lumen of the subject K, the pretest capsule 31 reaches the large intestine as the reach target region of the capsule endoscope. In this case, when the pretest capsule 31 reaches the small intestine, the pretest capsule 31 causes the small-intestine dissoluble unit 32b to be collapsed by the bile, thereby discharging the pigment 4. Thereafter, when the pretest capsule 31 reaches the large intestine, the pretest capsule 31 causes the large-intestine dissoluble unit 32a to be collapsed by a specific substance within the large intestine, thereby discharging the pigment 3. Therefore, when the pretest capsule 31 reaches the large intestine, the pigments 3 and 4 are excreted from the body together with the bodily waste of the subject K. On the other hand, when a stenosis part exists within the small intestine of the subject K, the pretest capsule 31 stagnates at this stenosis part within the small intestine, and causes the small-intestine dissoluble unit 32b to be collapsed by the bile, thereby discharging the pigment 4. In this case, the large-intestine dissoluble unit 32a stagnates without being dissolved (that is, the large-intestine dissoluble unit 32a does not reach the large intestine within a predetermined time), and therefore, the pigment 3 is not discharged. Accordingly, when the pretest capsule 31 reaches the small intestine, the pigment 4 out of two types of the pigments 3 and 4 is excreted from the body of the subject K together with his bodily waste. On the other hand, when there is a stenosis part (or a barrier) within esophagus or stomach of the subject K, the pretest capsule 31 stagnates there, and does not reach the small intestine and the large intestine. In this case, both the large-intestine dissoluble unit 32a and the small-intestine dissoluble unit 32b stagnate within the lumen without being dissolved, and therefore, the pigments 3 and 4 are not discharged. Therefore, when the pretest capsule 31 does not reach the small intestine, none of the pigments 3 and 4 is excreted together with the bodily waste of the subject K.

At Step S302, the user visually checks pigments within the bodily waste of the subject K (YES at Step S303). When the visually-checked pigments contained in the bodily waste have two colors (the pigments 3 and 4), (two colors at Step S304), the user determines, in a similar manner to that at Step S104 described above, that the pretest capsule 31 reaches the large intestine of the subject K within a predetermined time (Step S305). Based on a result of this determination, the user determines that when the subject K orally ingests the capsule endoscope, this capsule endoscope reaches the large intestine within the predetermined time by safely passing through the inside of the lumen (that is, there is no problem in the lumen passability of the capsule endoscope regarding the subject K). While it is described above that the user determines that the pretest capsule 31 has reached the large intestine when the visually-checked pigments in the bodily waste have two colors, the user can also determine that the pretest capsule 31 has reached the large intestine when the visually-checked pigments in the bodily waste have a mixed color (at least, a color different from the pigment 4) of the two colors (the pigments 3 and 4) not only the two colors.

Next, the user prompts the subject K to ingest a predetermined cleaning agent having no problem in the passability of the capsule endoscope through the lumen, thereby cleaning the inside of the small intestine and the large intestine of the subject K (Step S306). At Step S306, the pigments 3 and 4 and the rest of the outer case 32 remaining within the small intestine and the large intestine of the subject K are neutralized or removed from the body. For this cleaning agent, there can be listed a neutralizer of the pigments 3 and 4, a purgative drug, and an intestinal cleaning liquid. Thereafter, the user performs, in a similar manner to that at Step S106 described above, the capsule endoscope examination on the subject K whose inside of the small intestine and inside of the large intestine are cleaned (Step S307), and completes the process of checking a passability of the capsule endoscope through the lumen of the subject K.

On the other hand, when the user does not visually find any one of the pigments 3 and 4 in the bodily waste of the subject K at Step S302 (NO at Step S303), the user determines that the pretest capsule 31 does not reach the small intestine of the subject K within a predetermined time (Step S309). Based on a result of this determination, the user determines that a stenosis part exists within the lumen from esophagus to small intestine of the subject K, and determines that when the subject K orally ingests the capsule endoscope, the capsule endoscope stagnates at the stenosis part and does not easily reach even the small intestine within the predetermined time (that is, there is a problem in the passability of the capsule endoscope through the lumen of the subject K).

When the user determines that there is a problem in the passability of the capsule endoscope through the lumen of the subject K, the user prompts the subject K to orally ingest a dissolving liquid of the pretest capsule 31 in a similar manner to that at Step S108 (Step S310), thereby removing the pretest capsule 31 stagnated at the stenosis part within the lumen of the subject K.

Thereafter, the user determines, in a similar manner to that of Step S109 described above, not to perform the capsule endoscope examination on the subject K who has a problem in the passability of the capsule endoscope through the lumen (Step S311), and completes the process of checking a passability of the capsule endoscope through the lumen of the subject K.

On the other hand, when the user visually checks a pigment within the bodily waste of the subject K (YES at Step S303), and when the visually-checked pigment contained in the bodily waste has one color (the pigment 4 only), (one color at Step S304), the user determines that although the pretest capsule 31 reaches the small intestine of the subject K within a predetermined time, the pretest capsule 31 does not reach the large intestine (Step S308). Based on a result of this determination, the user determines that a stenosis part exists within the small intestine of the subject K, and that when the subject K orally ingests the capsule endoscope, the capsule endoscope stagnates at the stenosis part within the small intestine and does not easily reach the large intestine within a predetermined time (that is, there is a problem in the lumen passability of the capsule endoscope regarding the subject K). Thereafter, proceeding to Step S310 described above, the user performs the process procedure at Step S310 and onwards, and completes the process of checking a passability of the capsule endoscope through the lumen of the subject K.

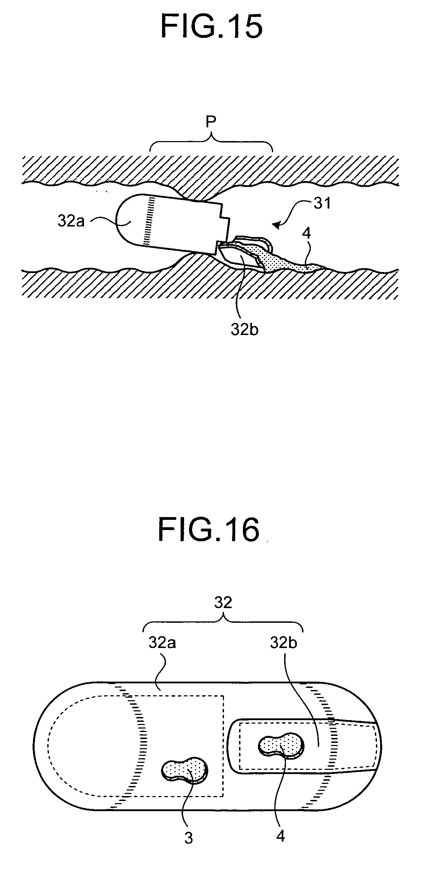

Next, discharge of the pigment 4 from the pretest capsule 31 stagnated at the stenosis part within the small intestine is described. FIG. 15 is a schematic diagram of a state that the pretest capsule 31 having stagnated at the stenosis part within the small intestine discharges the pigment 4. As shown in FIG. 15, when the stenosis part P smaller than an external diameter of the large-intestine dissoluble unit 32a (that is, an external diameter of the outer case 32) exists within the small intestine of the subject K, the advancement of the pretest capsule 31 is blocked by the stenosis part P of the small intestine, and stagnates there. The pretest capsule 31 having stagnated at the stenosis part P reaches the small intestine, but cannot reach the subsequent organ (that is, the large intestine).

The pretest capsule 31 having stagnated at the stenosis part P within the small intestine dissolves the small-intestine dissoluble unit 32b. In this case, the large-intestine dissoluble unit 32a is not dissolved by the bile within the small intestine, and maintains the current external shape. Therefore, as shown in FIG. 15, only the small-intestine dissoluble unit 32b is collapsed by the bile within the small intestine, and the pigment 4 is discharged to the inside of the small intestine by the collapse of the small-intestine dissoluble unit 32b. The pigment 4 discharged in this way is excreted from the body together with the bodily waste of the subject. Chitin or the like as strong alkali is preferable for the small-intestine dissoluble unit.

On the other hand, the large-intestine dissoluble unit 32a is not dissolved by the bile within the small intestine as described above, and therefore, maintains the current external shape and the external diameter of the large-intestine dissoluble unit 32a (an external diameter equivalent to that of the capsule medical device). Accordingly, the large-intestine dissoluble unit 32a cannot pass through (that is, being stagnated) the stenosis part P within the small intestine, and cannot reach the large intestine within a predetermined time. Consequently, the pigment 3 contained in the large-intestine dissoluble unit 32a is not discharged from the large-intestine dissoluble unit 32a, and is not excreted together with the bodily waste of the subject.

As described above, in the third embodiment of the present invention, the pretest capsule has an outer structure formed by connecting a small-intestine dissoluble unit that is dissolved by a strong alkali substance such as the bile within the small intestine, at one end of a large-intestine dissoluble unit having substantially a capsule shape that is dissolved by a specific substance within the large intestine. The outer structure includes therein two types of visually checkable identifiers having pigments of two colors. The outer structure discharges one identifier out of the two types of identifiers along the dissolution of the small-intestine dissoluble unit, and discharges the other identifier out of the two types of identifiers along the dissolution of the large-intestine dissoluble unit. Other configurations are substantially identical to those of the first embodiment. Accordingly, it is possible to check whether the outer structure has reached plural regions (small intestine and large intestine, for example) within the whole lumen from esophagus to large intestine. Based on this, it is possible to test whether the capsule medical device reaches the plural regions within the whole lumen from esophagus to large intestine. Consequently, there is an operational effect similar to that of the first embodiment, and it is possible to estimate a region (an organ) where a stenosis part exists, and check in detail a passability of the capsule medical device through the lumen. When the subject takes in moisture or an isotonic solution (a so-called intestinal lavage) that is not easily absorbed by a living body in advance, pigments can be easily excreted, and check can be performed more securely.

In the third embodiment of the present invention, while the small-intestine dissoluble unit 32b is connected to one end of the large-intestine dissoluble unit 32a to form a dome shape at one end of the capsule shape of the outer case 32, alternatively, the small-intestine dissoluble unit 32b can be connected to one end of the large-intestine dissoluble unit 32a to form one part within one end of the capsule shape of the outer case 32. Specifically, as shown in FIG. 16, the small-intestine dissoluble unit 32b can be formed in a structure (a U-shaped structure, for example) having a smaller width than that of the external diameter of the outer case 32 (that is, the external diameter of the large-intestine dissoluble unit 32a), and connected to one end of the large-intestine dissoluble unit 32a to form one part within one end of the capsule shape of the outer case 32. In this case, the pigment 3 is filled in the internal space of the large-intestine dissoluble unit 32a, and the pigment 4 is filled in the closed space encircled by an external wall of a recess-shaped end of the large-intestine dissoluble unit 32a and an internal wall of the small-intestine dissoluble unit 32b. When the width of the small-intestine dissoluble unit 32b is set smaller than the external diameter of the outer case 32 in this way, even after the small-intestine dissoluble unit 32b is collapsed, the large-intestine dissoluble unit 32a can maintain the external diameter equal to that of the capsule medical device and can maintain the capsule shape similar to the external shape of the capsule medical device. As a result, a passability of the capsule medical device through the lumen of the subject can be checked in a state nearer to a state that the capsule medical device is actually inserted into the body of the subject.

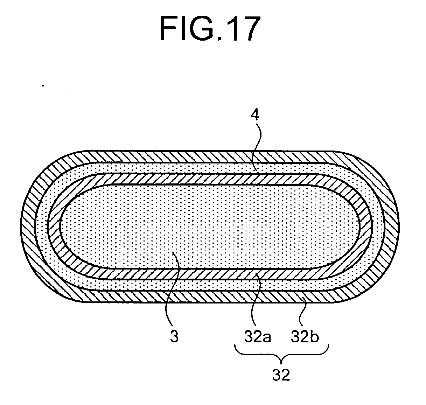

Further, in the third embodiment of the present invention, while the outer case 32 is formed by connecting the small-intestine dissoluble unit 32b to one end of the large-intestine dissoluble unit 32a, alternatively, the small-intestine dissoluble unit 32b can be arranged at the outside of the small-intestine dissoluble unit 32b, thereby forming the outer case 32 having a two-layer configuration. Specifically, as shown in FIG. 17, the small-intestine dissoluble unit 32b as a structure having a capsule shape similar to that of the large-intestine dissoluble unit 32a can be arranged at the outside of the large-intestine dissoluble unit 32a as a capsule-shaped structure, thereby providing the outer case 32. In this case, the large-intestine dissoluble unit 32a is covered by the small-intestine dissoluble unit 32b having this capsule shape. The pigment 3 is contained in the large-intestine dissoluble unit 32a within the small-intestine dissoluble unit 32b, and the pigment 4 is filled in the closed shape encircled by an external wall of the large-intestine dissoluble unit 32a having the capsule shape and an internal wall of the small-intestine dissoluble unit 32b having the capsule shape. In this way, even after the small-intestine dissoluble unit 32b at the outside is collapsed, the outer case 32 having the two-layer configuration of the large-intestine dissoluble unit 32a and the small-intestine dissoluble unit 32b can maintain the capsule shape similar to that of the capsule medical device. As a result, a passability of the capsule medical device through the lumen of the subject can be checked in a state nearer to a state that the capsule medical device is actually inserted into the body of the subject.

Further, in the first to third embodiments of the present invention and modifications thereof, the external shape of the outer structure is a capsule shape similar to that of the capsule medical device. Alternatively, the outer structure can have a desired external shape such as a spherical shape so long as the outer structure has an external diameter substantially equal to that of the capsule medical device.

In the first to third embodiments of the present invention and modifications thereof, pigments presenting always predetermined colors are used as visually checkable identifiers. Alternatively, the visually checkable identifiers contained in the outer structure can be reflection particles that reflect visible light, or can be transparent substances that generate a visually checkable change (visible in a predetermined color) by reacting with a substance within the large intestine (solid waste, for example), or can be transparent substances that generate a visually checkable change (visible in a predetermined color) by reacting with a predetermined reagent, or can be substances that generate a visually checkable change such as a shape change reacting with a substance within the large intestine or a predetermined reagent. The transparent substance can be a transparent and colorless substance. The visually checkable identifier can be a combination of these. When an identifier such as a transparent substance visually checkable after being excreted from the body is used, this identifier does not change the color within the lumen (particularly, the internal wall) of the subject. Therefore, it is not necessary to clean the inside of the intestines of the subject after checking the passability of the capsule medical device through the lumen. As a result, the subject does not have to orally ingest a cleaning agent to clean the inside of the intestines of the neutralizer or the like.

In the first to third embodiments of the present invention and modifications thereof, while a visually checkable identifier is used, alternatively, a liquid generating a strong smell or a gas generating a strong smell can be used (or generated), or these can be combined with a visually checkable identifier. Accordingly, a passability through the lumen can be more securely checked both visually and by smelling.

Further, in the first to third embodiments of the present invention and modifications thereof, while the reach target region of the capsule medical device is the large intestine, alternatively, the reach target region of the capsule medical device can be any organ within the digestive system from esophagus to large intestine. In this case, the outer structure containing the identifier to visually check whether the pretest capsule has reached the reach target region of the capsule medical device can be formed by using a member dissolved by a specific substance or pH existing in the organ as the reach target region.

In the first modification of the first embodiment of the present invention, the outer case 6 having the two-layer configuration of the internal dissoluble unit 6a dissolved by a specific substance within the large intestine and the external dissoluble unit 6b formed by a general enteric member is used. Alternatively, the thickness of the internal dissoluble unit 6a can be further increased, and an outer case of a one-layer configuration having the internal dissoluble unit 6a of the large thickness can be used as a large-intestine dissoluble layer. In this case, a thickness of the large-intestine dissoluble layer (the thick internal dissoluble unit 6a) can be set sufficient enough not to expose the inside pigment 3 by a substance of large-intestine origin flowing to the end of the small intestine.

Further, in the first to third embodiments of the present invention and modifications thereof, visually checkable identifiers such as pigments are filled in the outer structure. Alternatively, a contrast agent such as barium can be further filled in the outer structure, or a passive communication device such as an RF-ID tag can be further arranged. Alternatively, metal plating such as gold plating can be performed on the internal wall of the outer structure. With this arrangement, presence of a lumen passability checking device (a pretest capsule, for example) stagnated at a stenosis part within the lumen can be accurately detected by using an X-ray apparatus, an RF-ID communication apparatus, or a metal detector. As a result, a position of the stenosis part within the lumen can be accurately detected.

In the second embodiment of the present invention, the outer case 22 is formed by connecting the lids 22b and 22c that are dissolved when contacted to the body fluid within the lumen for a predetermined time or more, to both opening ends of the body unit 22a dissolved by a specific substance within the large intestine. Alternatively, an outer case as an outer structure of the pretest capsule can be formed by connecting lids that are dissolved by a specific substance within the large intestine to both opening ends of a body unit that is dissolved when contacted to the body fluid within the lumen for a predetermined time or more.

### INDUSTRIAL APPLICABILITY

As described above, the lumen passability checking device and the lumen passability checking method according to the present invention are useful to check beforehand a passability through the lumen of the capsule medical device to be inserted into the organ of the subject. Particularly, the invention is suitable for the lumen passability checking device and the lumen passability checking method capable of easily checking the passability of the capsule medical device through the lumen until when the capsule medical device reaches a target organ within the subject.

## Claims

1. A lumen passability checking device that checks a lumen passability of a capsule medical device to be inserted into a body of a subject, the lumen passability checking device comprising:
an outer structure having an external diameter substantially equal to that of the capsule medical device and having at least a part dissolved by a specific substance existing in a target organ within the subject; and
a visually checkable identifier contained in the outer structure and discharged from the outer structure to inside of the body of the subject along dissolution of the outer structure.

2. The lumen passability checking device according to claim 1, wherein the outer structure is collapsed by the specific substance.

3. The lumen passability checking device according to claim 1, wherein the outer structure comprises:
a first dissoluble unit dissolved by the specific substance; and
a second dissoluble unit dissolved by a substance in the body of the subject after a lapse of time longer than a time necessary for the capsule medical device to reach the target organ, wherein
the outer structure is shrink-deformed or collapsed by dissolution of the second dissoluble unit.

4. The lumen passability checking device according to claim 1, wherein the outer structure comprises:
a first dissoluble unit dissolved by the specific substance; and
a second dissoluble unit dissolved by another specific substance different from the specific substance, wherein
the outer structure is shrink-deformed or collapsed by dissolution of the second dissoluble unit.

5. The lumen passability checking device according to claim 1, further comprising another visually checkable identifier presenting a state different from that of the identifier, wherein
the outer structure comprises:
a first dissoluble unit containing the identifier and dissolved by the specific substance; and
a second dissoluble unit containing the another identifier and dissolved by another specific substance different from the specific substance, and wherein
the identifier is discharged from the outer structure to the inside of the body of the subject along dissolution of the first dissoluble unit, and the another identifier is discharged from the outer structure to the inside of the body of the subject along dissolution of the second dissoluble unit.

6. The lumen passability checking device according to claim 5, wherein the first dissoluble unit forms the external diameter of the outer structure and maintains the external diameter before and after dissolution of the second dissoluble unit.

7. The lumen passability checking device according to claim 1, wherein the identifier is a reflection particle that reflects visible light or a pigment.

8. The lumen passability checking device according to claim 1, wherein the identifier generates a visually checkable change by reacting with a substance within large intestine.

9. The lumen passability checking device according to claim 1, wherein the identifier generates a visually checkable change by reacting with a predetermined reagent.

10. The lumen passability checking device according to claim 8, wherein the identifier is substantially transparent having no color before reaction.

11. The lumen passability checking device according to claim 9, wherein the identifier is substantially transparent having no color before reaction.

12. A lumen passability checking method for checking a lumen passability of a capsule medical device to be inserted into a body of a subject, the method comprising:
ingesting for having the subject ingested a lumen passability checking device that includes a visually checkable identifier within an outer structure having an external diameter substantially equal to that of the capsule medical device and having at least a part dissolved by a specific substance existing in a target organ within the subject;
visually checking a bodily waste of the subject excreted during a period from when the subject ingests the lumen passability checking device until when a predetermined time elapses; and
determining that the capsule medical device passes through inside of the lumen of the subject and reaches the target organ, when the identifier is visually checked in the bodily waste of the subject at the visually checking.

13. The lumen passability checking method according to claim 12, wherein
at the ingesting, the subject ingests the lumen passability checking device that contains another identifier presenting a state different from that of the identifier in a dissoluble unit that is a part of the outer structure and is dissolved by a substance within the body different from the specific substance, and contains the identifier in a dissoluble unit that is a remaining part of the outer structure and is dissolved by the specific substance, and
at the determining, when the identifier and the another identifier are visually checked in the bodily waste of the subject at the visually checking, it is determined that the capsule medical device passes through inside of the subject and reaches the target organ.

14. The lumen passability checking method according to claim 13, wherein at the determining, when the another identifier is visually checked without visually checking the identifier at the visually checking, it is determined that the capsule medical device reaches the organ within the subject in which the substance within the subject exists.

15. The lumen passability checking method according to claim 12, wherein at the visually checking, a plurality of bodily wastes excreted from the subject are visually checked sequentially.

16. The lumen passability checking method according to claim 12, further comprising cleaning the inside of the lumen of the subject to which the identifier is discharged, when it is determined at the determining that the capsule medical device passes through the inside of the lumen of the subject and reaches the target organ.

17. The lumen passability checking method according to claim 12, further comprising dissolving-liquid ingesting at which the subject ingests a dissolving liquid for dissolving at least the part of the outer structure when it is determined at the determining that the capsule medical device does not reach the target organ.

18. A lumen passability checking method for checking a lumen passability of a capsule medical device to be inserted into a body of a subject, the method comprising:
ingesting for having the subject ingested a lumen passability checking device that includes an identifier within an outer structure having an external diameter substantially equal to that of the capsule medical device and having at least a part dissolved by a specific substance existing in a target organ within the subject;
visually checking for mixing a reagent into a bodily waste of the subject excreted during a period from when the subject ingests the lumen passability checking device until when a predetermined time elapses, thereby making the identifier in the bodily waste visible, and visually checking the visible identifier; and
determining that the capsule medical device passes through inside of the lumen of the subject and reaches the target organ, when the visible identifier is visually checked in the bodily waste of the subject at the visually checking.

19. The lumen passability checking method according to claim 18, further comprising dissolving-liquid ingesting at which the subject ingests a dissolving liquid for dissolving at least the part of the outer structure when it is determined at the determining that the capsule medical device does not reach the target organ.

20. A lumen passability checking device that checks a lumen passability of a capsule medical device to be inserted into a body of a subject, the lumen passability checking device comprising:
an outer structure having an external diameter substantially equal to that of the capsule medical device and having at least a part dissolved by a specific substance existing in a target organ within the subject; and
an identifier contained in the outer structure and discharged from the outer structure to inside of the body of the subject along dissolution of the outer structure, wherein
the identifier can be checked by a smell.

21. A lumen passability checking method for checking a lumen passability of a capsule medical device to be inserted into a body of a subject, the method comprising:
ingesting for having the subject ingested a lumen passability checking device that includes an identifier that can be checked by a smell within an outer structure having an external diameter substantially equal to that of the capsule medical device and having at least a part dissolved by a specific substance existing in a target organ within the subject;
checking presence of the smell of the identifier from a bodily waste of the subject excreted during a period from when the subject ingests the lumen passability checking device until when a predetermined time elapses; and
determining that the capsule medical device passes through inside of the lumen of the subject and reaches the target organ, when the smell of the identifier is checked from the bodily waste of the subject at the visually checking.
